# EUROPÄISCHE PATENTSCHRIFT

(11) **EP 0 520 263 B1**
(45) Veröffentlichungstag und Bekanntmachung des Hinweises auf die Patenterteilung: **25.10.1995**
(21) Anmeldenummer: 92109974.3
(22) Anmeldetag: 13.06.1992
(51) Int. Cl.: C07C 69/007, C07C 67/297

(54) **Verfahren zur Herstellung von 2-Methylenpropan-1,3-dioldicarboxylaten**
Process for the preparation of 2-methylenpropane-1,3-diol dicarboxylates
Procédé de préparation de dicarboxylates du 2-méthylènepropane-1,3-diol

(30) Priorität: 26.06.1991 DE 4121048
(43) Veröffentlichungstag der Anmeldung: 30.12.1992
(73) Patentinhaber: BASF Aktiengesellschaft, 67063 Ludwigshafen (DE)
(72) Erfinder: Merger, Franz, Dr., W-6710 Frankenthal (DE); Witzel, Tom, Dr., W-6700 Ludwigshafen (DE); Brudermüller, Martin, Dr., W-6800 Mannheim 1 (DE)

(56) Entgegenhaltungen:
- CHEMICAL ABSTRACTS, vol. 87, no. 7, 1977, Columbus, Ohio, US; abstract no. 52810, Seite 424 ;Spalte 1 ; & JP-A-77 027 710
- JOURNAL OF THE AMERICAN CHEMICAL SOCIETY Bd. 76, Nr. 9, 1954, WASHINGTON D.C., US Seiten 2285 - 2290 H E ZIMMERMAN ET AL 'The cleavage reaction of 1,3-diols'
- CHEMICAL ABSTRACTS, vol. 50, 1956, Columbus, Ohio, US; abstract no. 9288h, 'Degradation of some polymethylol derivatives'

## Beschreibung

Die vorliegende Erfindung betrifft ein Verfahren zur Herstellung von 2-Methylenpropan-1,3-dioldicarboxylaten (1,3-Diacyloxy-2-methylenpropanen) aus Pentaerythrittetracarboxylaten.

2-Methylenpropan-1,3-dioldicarboxylate sind vielseitig einsetzbare Synthesebausteine, u. a. wird das 2-Methylenpropan-1,3-dioldiacetat zur Herstellung von 2-Benzyl-4-hydroxymethylfuran (Elliott-Alkohol) eingesetzt (DE 35 46 371 A1, EP 187 345 A1). Auch zur Herstellung von Pyranen (THL, 28, 6219, 1987), von 1,3-Dihydroxyacetonderivaten (DE 25 56 525 A1) oder von 3,5-Dialkylpyridinen (Liebigs Annalen Chem., 111, 1973) sind sie einsetzbar.

In Bull. Soc. Chim. Fr. (1965), 1355 ist die Synthese des 2-Methylenpropan-1,3-diols aus Acrolein, Cyclopentadien und Formaldehyd über 2,2-Bis(hydroxymethyl)norbornen-5 beschrieben. Das gleiche Syntheseprinzip mit Anthracen statt Cyclopentadien ist aus FR 1350723 bekannt. Beide Verfahren verlaufen über eine dreistufige Reaktionssequenz: Diels-Alder-Reaktion, Aldol-Cannizarro- und Retro-Diels-Alder-Reaktion.

Die Oxidation von Isobuten in Gegenwart von Essigsäure an Pd-dotierten Al₂O₃-Katalysatoren erlaubt einen direkten Zugang zu 2-Methylenpropan-1,3-dioldiacetat (z.B. JP 52027710, JP 47028965, DE-OS 19 42 014, DE-OS 20 03 933). Auch Ester des Methallylalkohols können unter gleichen Bedingungen zu 2-Methylenpropan-1,3-dioldiacetat umgesetzt werden (z.B. DE-OS 20 54 987, DE-OS 19 09 964).

Es ist ebenfalls bekannt Methallylchlorid durch Chlorierung, Isomerisierung des entstehenden Olefingemisches an Zink(II)chlorid und Umsetzung mit Essigsäure/Triethylamin in das Diacetat zu überführen (DE 32 43 545 A1).

2-Methylenpropan-1,3-diol entsteht auch als Nebenprodukt bei der alkalisch katalysierten Dehydrobromierung von Pentaerythritmonobromid neben dem Hauptprodukt 3,3-Bis(hydroxymethyl)oxetan (Heterocycles, 14 (2), (1980), 189). Diese heterolytische Fragmentierung ist für 3-Halogenalkanole (Angew. Chem., 79 (1967), 1), besonders für 1,3-Diole (J. Am. Chem. Soc., 76 (1954), 2285) prinzipiell bekannt, präparativ aber nur begrenzt nutzbar, da die Ausbeuten zu gering sind.

Nachteile der bisher bekannten Verfahren zur Herstellung von 2-Methylenpropan-1,3-dioldiacetat sind daher neben der z.T. nur sehr geringen Ausbeute vor allem die vielstufige Reaktionsfolge der bekannten Verfahren, der hohe technische Aufwand, insbesondere wegen sicherheitstechnischer Probleme (Oxidation) oder die Verwendung von ungünstigen Ausgangsprodukten.

Der vorliegenden Erfindung lag daher die Aufgabe zugrunde, ein technisch einfach ausführbares Verfahren zur Herstellung von 2-Methylenpropan-1,3-dioldicarboxylaten zur Verfügung zu stellen, das die oben angegebenen Nachteile vermeidet.

Diese Aufgabe wurde gelöst durch ein Verfahren zur Herstellung von 2-Methylenpropan-1,3-dioldicarboxylaten der allgemeinen Formel I 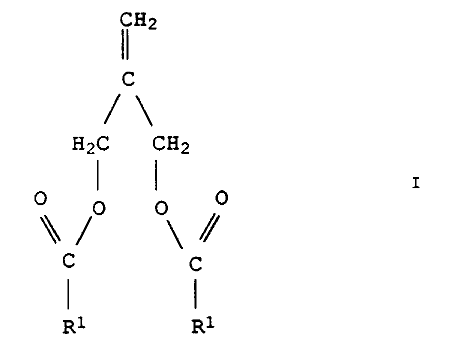
in der R¹ Wasserstoff oder ein Alkylrest mit 1 bis 4 C-Atomen ist, das dadurch gekennzeichnet ist, daß man Pentaerythrittetracarboxylate der allgemeinen Formel II 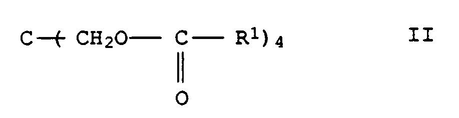
in der R¹ die oben angegebene Bedeutung hat, an Feststoffkatalysatoren bei Temperaturen von 150 bis 450°C, vorzugsweise 250 bis 400° und insbesondere bei 300 bis 350°C und Drücken von 1 mbar bis zu Normaldruck (Atmosphärendruck), vorzugsweise 50 bis 500 mbar, umsetzt.

Das Ausgangsprodukt der Formel II ist entweder als Handelsprodukt erhältlich oder kann in üblicher Weise durch Veresterung des Pentaerythritalkohols erhalten werden.

Die Pentaerythrittetracarboxylate werden vorzugsweise in der Gasphase mit dem Festkörperkatalysator in Kontakt gebracht, wobei die Reaktion diskontinuierlich oder insbesondere kontinuierlich durchgeführt wird. Die Reaktion kann in einem Festbett oder in einem Wirbelbett ausgeführt werden. Das Verfahren wird vorzugsweise unter vermindertem Druck durchgeführt.

Es kann auch unter Schutzgas, z.B. unter Stickstoff oder Argon, gearbeitet werden.

Bei der Umsetzung hält man vorteilhaft eine Katalysatorbelastung von 0,01 bis 20 g, insbesondere 0,1 bis 5 g Ausgangsstoff der Formel II je g Katalysator und Stunde ein.

Die Pentaerythrittetracarboxylate der Formel II können in den Reaktor als Schmelze oder gelöst in einem inerten Lösungsmittel eingebracht werden. Als inerte Lösungsmittel eignen sich beispielsweise Ether wie THF und Dioxan; aromatische Kohlenwasserstoffe wie Benzol, Toluol und Xylole; aliphatische Alkohole wie Ethanol, Methanol, n-Propanol und Isopropanol; aliphatische Carbonsäuren wie Essigsäure, Propionsäure und Buttersäure und Mischungen dieser Lösungsmittel.

Wird die Reaktion in einem Festbett durchgeführt, ist es bevorzugt, das Festbett vertikal auszurichten und über dem Katalysator inerte Materialien anzuordnen. Die Ausgangsprodukte werden dann von oben eingeführt und werden im Kontakt mit dem inerten Material auf Reaktionstemperatur gebracht.

Die Reaktionsprodukte werden in einer nachgeschalteten, gekühlten Vorlage aufgefangen. Die entstehenden Produkte werden z.B. durch Destillation aus dem Reaktionsgemisch isoliert; als Nebenprodukte treten u.a. Formaldehyd und die aus dem jeweiligen Ester abgespaltene Säure auf. Nicht umgesetzte Ausgangsstoffe können in die Umsetzung zurückgeführt werden.

Als Katalysatoren werden Feststoffkatalysatoren (Heterogen-Katalysatoren) eingesetzt. Geeignet sind z.B. Oxide von Elementen der dritten und vierten Hauptgruppe sowie der zweiten bis sechsten Nebengruppe des periodischen Systems. Bevorzugt sind Aluminiumoxide, Titanoxide, Zeolithe und/oder Heteropolysäuren, insbesondere des Wolframs und Molybdäns.

Es wird angenommen, daß insbesondere saure Gruppen an der Oberfläche der Katalysatoren für den Ablauf der Reaktion nützlich sind. Die Katalysatoren können in Form von gepreßten Strängen, aber auch in Kugelform, als Splitt oder als Pulver eingesetzt werden.

### Beispiele

### Beispiel 1

Eine Schmelze von 30 g Pentaerythrittetraacetat pro Stunde wurde bei 350°C und einem Druck von 50 bis 100 mbar auf eine Katalysatorschüttung bestehend aus 80 g Al₂O₃ (gepreßt als 1,5 mm-Stränge) und einer darüber angeordneten Schicht aus Quarzkugeln in einem beheizten Quarzrohr (Innendurchmesser 28 mm) aufgegeben. Die entstandenen gasförmigen Reaktionsprodukte wurden in einer nachgeschalteten, gekühlten Vorlage kondensiert.

Nach 1-stündigem Betrieb wurden 3,5 g 2-Methylenpropan-1,3-dioldiacetat isoliert.

Die Ausbeute betrug 21 % der Theorie, bezogen auf das eingesetzte Pentaerythrittetraacetat.

### Beispiel 2

Von einer Lösung von 31 Gew.-% Pentaerythrittetraacetat in Toluol wurde 70 g pro Stunde bei 300°C und einem Druck von 470 mbar auf eine Katalysatorschüttung bestehend aus 75 g Al₂O₃ (gepreßt als 1,5 mm-Stränge) und einer darüber angeordneten Schicht aus Quarzkugeln in einem beheizten Quarzrohr (Innendurchmesser 30 mm) aufgegeben. Nach 2-stündiger Laufzeit wurden 11 g 2-Methylenpropan-1,3-dioldiacetat kondensiert.

Die Ausbeute betrug 46 % der Theorie, bezogen auf das eingesetzte Pentaerythrittetraacetat.

### Beispiel 3

Von einer Lösung von 29 Gew.-% Pentaerythrittetraacetat in Essigsäure wurden 80 g pro Stunde bei 350°C und einem Druck von 170 mbar auf eine Katalysatorschüttung bestehend aus 260 g Al₂O₃ (gepreßt als 1,5-mm-Stränge) und einer darüber angeordneten Schicht aus Quarzkugeln in einem beheizten Quarzrohr (Innendurchmesser 50 mm) aufgegeben. Nach 5-stündiger Laufzeit wurden 25,7 g 2-Methylenpropan-1,3-dioldiacetat kondensiert.

Die Ausbeute betrug 38 % der Theorie, bezogen auf das eingesetzte Pentaerythrittetraacetat.

### Beispiel 4

Von einer Lösung von 30 Gew.-% Pentaerythrittetraacetat in Eisessig wurden 30 g pro Stunde bei 300°C und einem Druck von 400 mbar auf eine Katalysatorschüttung bestehend aus 260 g Al₂O₃ (als 1,5-mm-Splitt) und einer darüber angeordneten Schicht aus Quarzkugeln in einem beheizten Quarzrohr (Innendurchmesser 30 mm) aufgegeben. Nach 2-stündiger Laufzeit wurden 2,7 g 2-Methylenpropan-1,3-dioldiacetat kondensiert.

Die Ausbeute betrug 26 % der Theorie, bezogen auf das eingesetzte Pentaerythrittetraacetat.

### Beispiel 5

Von einer Lösung von 30 Gew.-% Pentaerythrittetraacetat in Eisessig wurden 42 g pro Stunde bei 350°C und einem Druck von 300 mbar auf eine Katalysatorschüttung, bestehend aus 62 g TiO₂ (als 1,5-mm-Stränge) und einer darüber angeordneten Schicht aus Quarzkugeln in einem beheizten Quarzrohr (Innendurchmesser 30 mm) aufgegeben. Laufzeit der Reaktion: 2 Stunden.

Die Ausbeute an 2-Methylenpropan-1,3-dioldiacetat betrug ca. 10 % der Theorie, bezogen auf das eingesetzte Pentaerythrittetraacetat.

## Patentansprüche

1. Verfahren zur Herstellung von 2-Methylenpropan-1,3-dioldicarboxylaten der allgemeinen Formel I 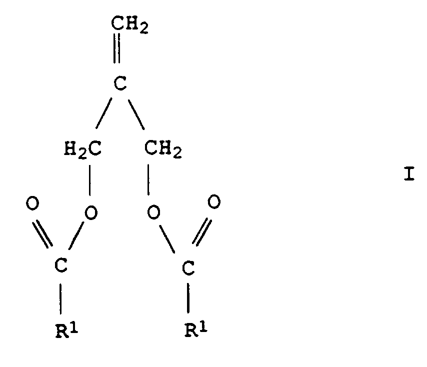 in der R¹ Wasserstoff oder ein Alkylrest mit 1 bis 4 C-Atomen ist, dadurch gekennzeichnet, daß man Pentaerythrittetracarboxylate der allgemeinen Formel II 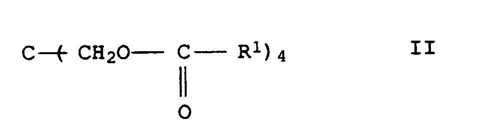 in der R¹ die oben angegebene Bedeutung hat, an Feststoffkatalysatoren bei Temperaturen von 150 bis 450°C und Drücken von 1 mbar bis zu Normaldruck umsetzt.

2. Verfahren nach Anspruch 1, dadurch gekennzeichnet, daß man die Umsetzung bei 250 bis 400°C und bei Drücken von 50 bis 500 mbar ausführt.

3. Verfahren nach Anspruch 1, dadurch gekennzeichnet, daß man als Feststoffkatalysatoren oxidische Feststoffkatalysatoren einsetzt.

4. Verfahren nach Anspruch 3, dadurch gekennzeichnet, daß man als Katalysatoren Aluminiumoxide, Titanoxide, Zeolithe oder Heteropolysäuren oder Gemische aus diesen Stoffen einsetzt.

5. Verfahren nach Anspruch 1, dadurch gekennzeichnet, daß R¹ eine Methylgruppe ist.

## Claims

1. A process for preparing 2-methylenepropane-1,3-diol dicarboxylates of the formula I 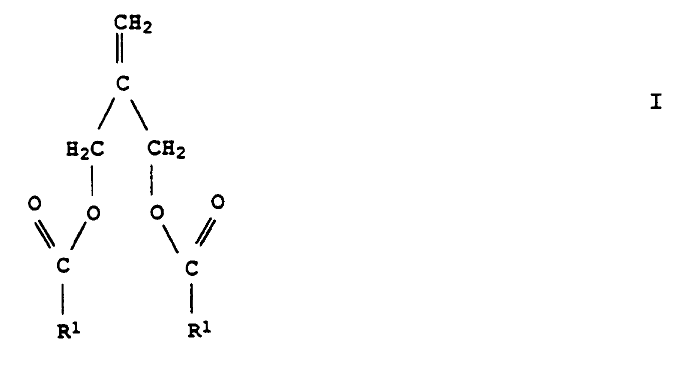 where R¹ is hydrogen or alkyl of 1 to 4 carbons, which comprises reacting pentaerythritol tetracarboxylates of the formula II 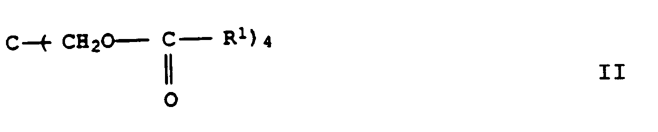 where R¹ has the abovementioned meaning, on solid catalysts at from 150 to 450°C and under from 1 mbar to atmospheric pressure.

2. A process as claimed in claim 1, wherein the reaction is carried out at from 250 to 400°C and under from 50 to 500 mbar.

3. A process as claimed in claim 1, wherein the solid catalysts are oxides.

4. A process as claimed in claim 3, wherein aluminum oxides, titanium oxides, zeolites or heteropolyacids or mixtures of these substances are employed as catalysts.

5. A process as claimed in claim 1, wherein R¹ is methyl.

## Revendications

1. Procédé de préparation de dicarboxylates de 2-méthylènepropane-1,3-diol de formule générale I 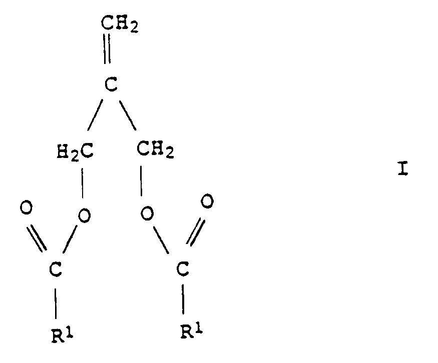 dans laquelle R¹ est un atome d'hydrogène ou un reste alkyle à 1-4 atomes de carbone, caractérisé en ce que l'on fait réagir des tétracarboxylates de pentaérythritol de formule générale II 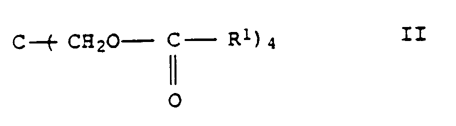 dans laquelle R¹ a la signification donnée ci-dessus, en présence de catalyseurs solides à des températures de 150 à 450°C et sous des pressions comprises entre 1 mbar et la pression normale.

2. Procédé selon la revendication 1, caractérisé en ce que l'on conduit la réaction à des températures de 250 à 400°C et sous des pressions de 50 à 500 mbar.

3. Procédé selon la revendication 1, caractérisé en ce que l'on utilise, comme catalyseurs solides, des catalyseurs solides à l'état oxydé.

4. Procédé selon la revendication 3, caractérisé en ce que l'on utilise, comme catalyseurs, des oxydes d'aluminium, des oxydes de titane, des zéolithes, des hérétopolyacides ou des mélanges de ces substances.

5. Procédé selon la revendication 1, caractérisé en ce que R¹ est un groupement méthyle.
